# EUROPÄISCHE PATENTANMELDUNG

(11) **EP 2 154 126 A1**
(43) Veröffentlichungstag der Anmeldung: **17.02.2010**
(21) Anmeldenummer: 08162024.7
(22) Anmeldetag: 07.08.2008
(51) Int. Cl.: C07C 253/34, C07C 255/08

(54) **Verfahren zur Aufreinigung von Acrylnitril in Kolonnen mit beschichteten Kolonnenböden**

(71) Anmelder: Ineos Manufacturing Deutschland GmbH, 50769 Köln (DE)
(72) Erfinder: Ritter, Frank, 41541 Dormagen (DE)
(74) Vertreter: Blum, Erwin

(57) **Zusammenfassung**

Die Erfindung betrifft ein Verfahren zur Aufreinigung von Acrylnitril aus einem Acrylnitril enthaltenden Ausgangsprodukt, wobei das Acrylnitril enthaltende Ausgangsprodukt in Kolonnen einer Destillation unterworfen wird, wobei die Kolonnen mit Gasdurchtrittslöchern versehene Kolonnenböden aufweisen, wobei die Kolonnenböden folgende Beschichtung aufweisen: I eine Grundierung enthaltend ein Tetrafluorpolymer/Bindeharz-Gemisch; II eine Schicht enthaltend ein Tetrafluorpolymer.

## Beschreibung

Die Erfindung betrifft Verfahren zur Aufreinigung von Acrylnitril aus einem Acrylnitril enthaltenden Ausgangsprodukt, wobei das Acrylnitril enthaltende Ausgangsprodukt in Kolonnen einer Destillation unterworfen wird, wobei die Kolonnen mit Gasdurchtrittslöchern versehene Kolonnenböden aufweisen, und wobei die Kolonnenböden beschichtet sind.

**Technischer Hintergrund der Erfindung**

Die vorliegende Erfindung setzt bei der Herstellung von Acrylnitril ein, insbesondere bei der Aufreinigung von Acrylnitril, speziell bei der Trennung der Blausäure aus dem Acrylnitril/Blausäure-Gemisch und der weiteren Aufreinigung des reinen Acrylnitrils.

Aus dem in vorlaufenden Verfahren synthetisierten Rohnitril wird im Destillations-Abschnitt Rein-Acrylnitril und Blausäure gewonnen. Von dem in der Scheideflasche der Rohnitrilkolonne anfallenden Rohnitril wird in der Blausäure-Acrylnitrilkolonne die Blausäure durch Destillation abgetrennt.

Die Blausäure wird zur Stabilisierung und Lagerung abgegeben. Der wasserhaltige, blausäurefreie Sumpf der Kolonne wird zur Entwässerung in die Trockenkolonne gefahren. Dort wird unter Ausnutzung des Acrylnitril-Wasser-Azeotrops das Wasser über den Kopf der Kolonne ausgekreist.

Leichtsiedende Verunreinigungen werden mit dem abgeschiedenen Wasser in den Waschkreislauf zurückgefahren. Der Sumpf, der nur Acrylnitril und hochsiedende Verunreinigungen enthält, wird in der Acrylnitrilreinkolonne weiter durch Destillation gereinigt. Über einen Seitenstrom wird das Acrylnitril als Ware abgezogen. Es wird in Tanks aufgefangen, stabilisiert und nach erfolgter Analyse zum Haupttanklager abgepumpt.

Im Kopf der Acrylnitrilreinkolonne sammeln sich noch geringe Mengen leichtsiedender Verunreinigungen, daher wird ständig etwas Kopfprodukt vor die Trockenkolonne zurückgeschleust. Die hochsiedenden Verunreinigungen werden aus dem Sumpf zusammen mit Acrylnitril ausgeschleust und zur weiteren Verarbeitung geleitet.

Die Kolonnenböden der Acrylnitril-Blausäure-Kolonnen und der nachgeschalteten Acrylnitril-Kolonnen verschmutzen während des Produktionsprozesses durch eine unerwünschte aber nicht vollständig vermeidbare Polymerisation. Das Polymerisat haftet an den Kolonnenböden. Dies führt zum Druckanstieg und somit zur Abstellung und Wartung der Kolonnen. Bei der Wartung werden die Kolonnenböden demontiert, gereinigt und in die ebenfalls gereinigte Kolonne wieder eingebaut.

Die Aufgabe der Erfindung war es daher, die Verschmutzung der Kolonnenböden zu verringern und dadurch die Wartungsintervalle zu verlängern und so Kosten einzusparen, sowie den Produktionsausfall zu vermindern.

Die technische Aufgabe wurde gelöst durch ein Verfahren zur Aufreinigung von Acrylnitril aus einem Acrylnitril enthaltenden Ausgangsprodukt, wobei das Acrylnitril enthaltende Ausgangsprodukt in Kolonnen einer Destillation unterworfen wird, wobei die Kolonnen mit Gasdurchtrittslöchern versehene Kolonnenböden aufweisen, wobei die Kolonnenböden folgende Beschichtung aufweisen: I eine Grundierung enthaltend ein Tetrafluorpolymer/Bindeharz-Gemisch; II eine Schicht enthaltend ein Tetrafluorpolymer. Dabei handelt es sich bei der Schicht I ₂₅ um eine Grundierungsschicht (oder Primer genannt), auf die die Schicht II mittelbar oder unmittelbar aufgelagert ist. Auf der Schicht II können weitere Fluorpolymer enthaltende Schichten aufgetragen sein.

In einem bevorzugten Verfahren wird als Schicht I ein Polytetrafluorethylen (PTFE)/Bindeharz-Gemisch verwendet. Weiterhin ist bevorzugt, dass in Schicht II ein Polytetrafluorethylen (PTFE) und/oder Perfluor-Alkoxy-Polymer (PFA) als Tetrafluorpolymer verwendet wird, vorzugsweise ein Perfluor-Alkoxy-Polymer.

In einem weiteren bevorzugten Verfahren wird als Schicht I ein Polytetrafluorethylen (PTFE)/Bindeharz-Gemisch und in Schicht II ein Perfluor-Alkoxy-Polymer (PFA) als Tetrafluorpolymer verwendet.

Die verwendeten Kolonnenböden bestehen dabei bevorzugt aus Metall, bevorzugt Stahl, besonders bevorzugt aus Edelstahl.

In einem bevorzugten Verfahren ist das Bindeharz in der Grundierungsschicht I anorganisch pigmentiert. Dabei können der Beschichtung Pigmente in einer Menge von 0,5 bis 5 Gewichtsprozent zugesetzt werden.

Weiterhin ist bevorzugt, dass in der Schicht II das Tetrafluorpolymer, vorzugsweise das Polytetrafluorethylen (PTFE) und/oder das Perfluor-Alkoxy-Polymer (PFA), im Gemisch mit Mineralteilchen vorliegt. Ganz besonders bevorzugt wird Perfluor-Alkoxy-Polymer (PFA) im Gemisch mit Mineralteilchen in Schicht II verwendet. So hat sich herausgestellt, dass die Durchlässigkeit des Schichtverbundes besonders vorteilhaft verändert wird, wenn mindestens die zweite Schicht zusätzlich mit anorganischen Zusätzen versehen wird. Es kann eine Zugabe von Mineralteilchen von 5 bis 25 Gewichtsprozent in die jeweilige Schicht erfolgen. Dabei wird davon ausgegangen, dass diese Mineralteilchen die Schicht armieren, was zu einer Erhöhung der mechanischen und thermischen Festigkeit und zu einer Verringerung der Permeabilität führt. In Versuchen hat sich gezeigt, dass die Menge der Polymerablagerungen und deren Affinität zur Beschichtung noch geringer waren, wenn in der Schicht II Mineralteilchen vorhanden waren.

In einem weiteren bevorzugten Verfahren sind die Mineralteilchen aus der Schicht II Glimmerteilchen. Diese Partikel verleihen der Schicht vorteilhafte Eigenschaften. Sie erhöhen nämlich die mechanische und thermische Festigkeit der Schicht, sie verringern jedoch vor allem die Permeabilität, indem sie den Permeationsweg verlängern. Die Stärke der Mineralteilchen (bevorzugt Glimmerteilchen) beträgt dabei von 1 bis 20 µm, vorzugsweise von 2 bis 10 µm, und der Durchmesser von 20 bis 300 µm, vorzugsweise von 50 bis 150 µm. Die Glimmerteilchen haben vorzugsweise eine Stärke von 2 bis 10 µm und einen Durchmesser bis zu 150 µm.

Weiterhin handelt es sich bevorzugt um ein Verfahren zur Aufreinigung von Acrylnitril aus einem Acrylnitril/Blausäure-Gemisch, d.h. die Kolonnenböden, die wie oben erläutert beschichtet sind, werden in Acrylnitril/Blausäure-Kolonnen verwendet.

In einer anderen Ausführungsform handelt es sich bevorzugt um ein Verfahren zur Aufreinigung von Acrylnitril aus einem vorgereinigten Acrylnitril, dem bereits die Blausäure entzogen wurde. Dies bedeutet, dass auch die Kolonne, die der Acrylnitril/Blausäure-Kolonne nachgeschaltet ist, mit Kolonnenböden ausgestattet werden kann, die wie oben erwähnt, beschichtet sind.

Die technische Aufgabe wird weiterhin gelöst durch die Verwendung von Kolonnenböden, die eine Beschichtung aufweisen enthaltend I eine Grundierung enthaltend ein Tetrafluorpolymer/Bindeharz-Gemisch; II eine Schicht enthaltend Tetrafluorpolymer, vorzugsweise ein Polytetrafluorethylen (PTFE) und/oder ein Perfluor-Alkoxy-Polymer (PFA), in Kolonnen, in denen eine Destillation von Acrylnitril aus einem Acrylnitril enthaltenden Ausgangsprodukt durchgeführt wird.

Die Erfindung stellt zudem die Verwendung von Kolonnenböden mit folgender Beschichtung I eine Grundierung enthaltend ein Tetrafluorpolymer/Bindeharz-Gemisch; II eine Schicht enthaltend Tetrafluorpolymer, vorzugsweise ein Polytetrafluorethylen (PTFE) und/oder ein Perfluor-Alkoxy-Polymer (PFA), in Kolonnen, in denen eine Destillation von Acrylnitril aus einem Acrylnitril enthaltenden Ausgangsprodukt durchgeführt wird, zur Verringerung der Anhaftung von Polymer an den genannten Kolonnenböden.

Die bevorzugten Ausführungsformen wurden bereits oben erläutert.

Das Grundmaterial wird vorteilhafterweise vor dem Aufbringen der ersten Schicht einer Sandstrahl- und/oder Flammspritzbehandlung unterzogen. Zur Verbesserung der Haftung des Schichtverbundes auf dem Grundmaterial wird eine erste Schicht I als Grundierung oder Primer aufgebracht. Diese Grundierung kann vorzugsweise aus einer Schicht aus Polytretrafluorethylen (PTFE) bestehen.

### Beispiele

Zur Erprobung welche Beschichtungen der Kolonnenböden ein Anhaften des gebildeten Polymers verringern bzw. verhindern, wurden mehrere Versuche mit kleineren Probestücken aus Edelstahl, die mit diversen Polytetrafluorethylen (PTFE) bzw. Perfluor-Alkoxy-Polymer (PFA) Beschichtungen versehen wurden, in den Acrylnitril-Blausäure-Kolonnen gefahren.

Diese Probestücke wurden in den Kolonnen auf einem der Kolonnenböden verschraubt. Ziel war es, eine beständige Beschichtung zu finden, welche das entstehende Polymer nicht auf der Beschichtung anhaften ließ. Diese Versuche mit einer Laufzeit von mehreren Monaten zeigten, dass die PTFE-Beschichtungen nicht beständig gegen das Produkt waren (Blasenbildung und Ablösung der Beschichtung), bzw. auch eine Anhaftung nicht verhindern konnten. Die Versuche beinhalteten auch Überlegungen anstelle von glatter Oberflächenstruktur, eine raue PTFE-Beschichtung zu versuchen. Diese raue PTFE-Beschichtung hatte zwar teilweise die benötigte Produktbeständigkeit, jedoch verhinderte sie nicht die Polymeranhaftung.

In einem weiteren Versuch wurden nochmals drei verschiedene PTFE-Beschichtungen auf dem 10. Boden der Kolonne montiert. Die Ergebnisse werden in der nachfolgenden Tabelle zusammengefasst.

**Tabelle: Ergebnis verschiedener Beschichtungen**

| **Nr.** | **Aufbau (Erscheinung)** | **Beobachtung,** **Ergebnis** | **erfindungsgemäß/ Vergleichsbeispiel** |
|---|---|---|---|
| **1** | i) Bindeharz-PTFE-Gemisch, ii) PTFE (ohne Mineral) (hellgrün, glatte Oberfläche) | beständig, leichte Polymeranbackungen, Polymer noch "abwischbar" | erfindungsgemäß |
| **2** | i) Bindeharz-PTFE-Gemisch, ii) PFA-Mineral-Gemisch (Mineral = Glimmer); (grün, glatte Oberfläche) | beständig, kaum Polymerablagerung, leichte Blauverfärbung, Polymer leicht "abwischbar" | erfindungsgemäß |
| 3 | einfache PTFE-Schicht; (gelb, glatt bis leicht raue Oberfläche) | nicht beständig, Blasenbildung, Polymerablagerungen | Vergleichsbeispiel |

Angesichts dieses Resultats wurden zwei Kolonnenböden (halbmondförmige Edelstahlbleche mit den Abmessungen 1040 mm x 385 mm x 2mm) mit den PTFE-Beschichtungen Nr. 1 und Nr. 2 auf den 10. und 9. Boden der Kolonne als nächster Kleinversuch montiert.

### Ergebnis:

Beide Beschichtungen waren auch mit großer Oberfläche produktbeständig. Die Beschichtung Nr. 1 hatte gegenüber der Beschichtung Nr. 2 mehr Polymerablagerungen (sowie auch Anbackungen in den Poren), die schwerer "abwischbar" waren.

Die Beschichtung Nr. 2 hatte wenige Polymerablagerungen, die leicht von Hand "abwischbar" waren, sowie eine leichte Blauverfärbung. Der für diese Beschichtung erforderliche Primer (Grundierung) enthält ein anorganisch pigmentiertes Bindeharz/PTFE - Gemisch, welches die Haftung zum Untergrund verbessert. Da die klare Deckschicht mikroporös ist, ist es möglich, dass Produkte diese Schicht durchwandern und mit dem Pigment reagieren. Es wird vermutet, dass sich das Cr(III)-oxid Pigment zum schwerlöslichen Hydroxid umgewandelt hat. Dies hatte jedoch keine Auswirkungen auf das Produkt bzw. auf die Beschichtung, sondern nur auf die Farbe der Beschichtung.

Anschließend wurde ein Großversuch mit 10 Böden und der Beschichtung Nr. 2 durchgeführt. Die Kolonnenböden wurden allseitig ummantelt.

### Ergebnis:

Die Laufzeit der Kolonne betrug 7,5 Monate (Durchschnitt 4,75 Monate) bedingt durch geplante Abstellung der Gesamtanlage. Die Kolonne hätte auch noch weiter betrieben werden können. Nach Kondensatspülung konnte der 10 Boden begutachtet werden, Ablagerungen waren nicht ersichtlich. Auch ein "Ausdämpfen" mit 5 bar Dampf gegen die Atmosphäre bei ca. 150 °C hat keinen Einfluss auf die Haltbarkeit der Beschichtung.

## Patentansprüche

1. Verfahren zur Aufreinigung von Acrylnitril aus einem Acrylnitril enthaltenden Ausgangsprodukt, wobei das Acrylnitril enthaltende Ausgangsprodukt in Kolonnen einer Destillation unterworfen wird, wobei die Kolonnen mit Gasdurchtrittslöchern versehene Kolonnenböden aufweisen, **dadurch gekennzeichnet, dass** die Kolonnenböden folgende Beschichtung aufweisen: I eine Grundierung enthaltend ein Tetrafluorpolymer/Bindeharz-Gemisch; II eine Schicht enthaltend ein Tetrafluorpolymer.

2. Das Verfahren nach Anspruch 1, **dadurch gekennzeichnet, dass** als Schicht I ein Polytetrafluorethylen (PTFE)/Bindeharz-Gemisch verwendet wird.

3. Das Verfahren nach Anspruch 1 oder 2, **dadurch gekennzeichnet, dass** in Schicht II ein Polytetrafluorethylen (PTFE) und/oder Perfluor-Alkoxy-Polymer (PFA) als Tetrafluorpolymer verwendet wird, vorzugsweise Perfluor-Alkoxy-Polymer.

4. Das Verfahren nach irgend einem der Ansprüche 1 bis 3, **dadurch gekennzeichnet, dass** das Bindeharz in der Grundierungsschicht I anorganisch pigmentiert ist.

5. Das Verfahren nach irgend einem der Ansprüche 1 bis 4, **dadurch gekennzeichnet, dass** in der Schicht II das Tetrafluorpolymer, vorzugsweise das Polytetrafluorethylen (PTFE) und/oder das Perfluor-Alkoxy-Polymer (PFA), im Gemisch mit Mineralteilchen vorliegt.

6. Das Verfahren nach Anspruch 5, **dadurch gekennzeichnet, dass** als Mineralteilchen Glimmerteilchen verwendet werden.

7. Das Verfahren nach irgend einem der Ansprüche 1 bis 6, **dadurch gekennzeichnet, dass** das Acrylnitril enthaltende Ausgangsprodukt ein Acrylnitril/Blausäure-Gemisch ist.

8. Das Verfahren nach irgend einem der Ansprüche 1 bis 7, **dadurch gekennzeichnet, dass** das Acrylnitril enthaltende Ausgangsprodukt ein bereits vorgereinigtes Acrylnitril darstellt, dem bereits die Blausäure entzogen wurde.

9. Verwendung von Kolonnenböden, die eine Beschichtung aufweisen enthaltend I eine Grundierung enthaltend ein Tetrafluorpolymer/Bindeharz-Gemisch, vorzugsweise ein Polytetrafluorethylen (PTFE)/Bindeharz-Gemisch; II eine Schicht enthaltend Tetrafluorpolymer, vorzugsweise ein Polytetrafluorethylen (PTFE) und/oder ein Perfluor-Alkoxy-Polymer (PFA), in Kolonnen, in denen eine Destillation von Acrylnitril aus einem Acrylnitril enthaltenden Ausgangsprodukt durchgeführt wird.

10. Verwendung von Kolonnenböden mit folgender Beschichtung I eine Grundierung enthaltend ein Tetrafluorpolymer/Bindeharz-Gemisch, vorzugsweise ein Polytetrafluorethylen (PTFE)/Bindeharz-Gemisch; II eine Schicht enthaltend Tetrafluorpolymer, vorzugsweise ein Polytetrafluorethylen (PTFE) und/oder ein Perfluor-Alkoxy-Polymer (PFA), in Kolonnen, in denen eine Destillation von Acrylnitril aus einem Acrylnitril enthaltenden Ausgangsprodukt durchgeführt wird, zur Verringerung der Anhaftung von Polymer an den genannten Kolonnenböden.
